# EUROPEAN PATENT APPLICATION

(11) **EP 2 177 216 A1**
(43) Date of publication of application: **21.04.2010**
(21) Application number: 08380291.8
(22) Date of filing: 13.10.2008
(51) Int. Cl.: A61K 31/34, A61P 17/02

(54) **Use of dianhydrohexite mononitrate derivatives as healing agents**

(71) Applicant: LACER, S.A., 08025 Barcelona (ES)
(72) Inventor: Mourelle Mancini, Marisabel, 08025 Barcelona (ES); Del Castillo Nieto, Juan Carlos, 08025 Barcelona (ES); Pubill Coy, Francisco, 08025 Barcelona (ES); Repollés Moliner, José, 08025 Barcelona (ES)
(74) Representative: ABG Patentes, S.L.

(57) **Abstract**

The present invention relates to the use of a compound of formula (I) or a tautomer, a pharmaceutically acceptable salt, a prodrug or a solvate thereof: 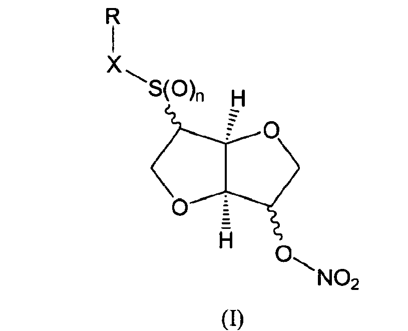 as active ingredient(s) in the manufacture of a pharmaceutical composition to heal wounds.

## Description

### FIELD OF THE INVENTION

The present invention relates to the healing of wounds.

### BACKGROUND

The primary goal in the treatment of wounds is to achieve wound closure. Open cutaneous wounds represent one major category of wounds and include burn wounds, neuropathic ulcers, pressure sores, venous stasis ulcers, and diabetic ulcers. Open cutaneous wounds routinely heal by a process which comprises six major components: i) inflammation, ii) fibroblast proliferation, iii) blood vessel proliferation, iv) connective tissue synthesis v) epithelialization, and vi) wound contraction. Wound healing is impaired when these components, either individually or as a whole, do not function properly. Numerous factors can affect wound healing, including malnutrition, infection, pharmacological agents (e.g., actinomycin and steroids), diabetes, and advanced age [Hunt and Goodson in Current Surgical Diagnosis & Treatment (Way; Appleton & Lange), pp. 86-98 (1988)].

With respect to diabetes, it is known that delayed wound healing causes substantial morbidity in patients suffering from that illness. Diabetes mellitus is a chronic disorder of glucose metabolism and homeostasis that damages many organs. It is the eighth leading cause of death in the United States (Harris et al., Diabetes 36:523 (1987)). Patients suffering from diabetes, vascular disease, neuropathy, infections, and recurrent trauma predispose the extremities, especially the foot, to pathologic changes. These pathological changes can ultimately lead to chronic ulceration, which may necessitate amputation.

Several pharmaceutical modalities have also been utilized in an attempt to improve wound healing. For example, treatment regimens involving zinc sulfate have been utilized by some practitioners. However, the efficacy of these regimens has been primarily attributed to their reversal of the effects of sub-normal serum zinc levels (e.g., decreased host resistance and altered intracellular bactericidal activity) [Riley, Am. Fam. Physician 24:107 (1981)]. While other vitamin and mineral deficiencies have also been associated with decreased wound healing (e.g., deficiencies of vitamins A, C and D; and calcium, magnesium, copper, and iron), there is no strong evidence that increasing the serum levels of these substances above their normal levels actually enhances wound healing. Thus, except in very limited circumstances, the promotion of wound healing with these agents has met with little success.

What is needed is a safe, effective, and interactive means for enhancing the healing of wounds of all types and without regard to the type of wound or the nature of the patient population to which the subject belongs.

Nitric oxide (NO) has also been shown to have a direct or an indirect role in pathophysiology of numerous bodily functions both in human and mammals. Some of these bodily functions and disorders include but not limited to (1) blood flow and pressure in body circulatory system, (2) pulmonary hypertension, (3) asthma, (4) inflammatory response, (5) infection, (6) cancer, (7) angiogenesis, (8) neurotransmission in nervous system, (9) diabetes, and (10) sexual dysfunction such as penile erection. Over the past several years, NO has also been noted to play an important role in wound healing. (Soneja, A et al. Pharmacol. Reports 57:108-119 (2005)

Conventionally wounds heal through a three step process. The first step is called an initial inflammatory phase. This phase is defined by platelet aggregation, degranulation, and phagocytosis.

The second step is referred to as the proliferative phase. This phase is characterized by an expansion of reparative cells. The reparative cells include fibroblasts. Fibroblasts are a major synthetic element in a wound, and are responsible for production and reorganization of structural proteins (such as collagen) required for tissue repair. Endothelial migration and angiogenesis also initiate in this stage.

The third and last step is called the maturation phase. This phase is the longest stage in the wound healing process. In this phase, newly deposited collagen (from fibroblasts) and an extracellular matrix are reorganized and result in increasing wound strength and eventually in mature scar formation.(Ziche et al. J. Neurooncology 50:139-148 (2000).

NO is both directly and indirectly, as a regulator, involved in each of these physiological steps. (Garg et al. Journal of Clinical Invest 83:1774-1777 (1989) De Graaf et al Circulation 85:2284-90 (1992) Radomsky et al. Brit. J of Pharmacol 92 :181-187 (1987) Sitzges et al. Biochem Biophys Res Comm 1192:1198-1203 (1993) Lefer et al. Circulation 88:2337-2350 (1993) Loscalzo et al. Clin Appl Throm Haemost 2:7-10 (1996) Moro et al. Pnas 93:1480-1485 (1996) In fact, many wound resident cells have the ability to synthesize or affect the synthesis of NO. Examples of wound resident cells include and are not limited to macrophages, neutrophiles, endothelial cells, vascular smooth muscle cells, keratinocytes, lymphocytes, and fibroblasts. Lack of NO and arginine in mammals result in a decrease in (a) NO metabolism, (b) wound breaking strength, (c) collagen synthesis, (d) epithelialization, and (e) wound contraction. (Seifter et al. Surgery 84 :224-230 (1978) In complementary studies that used chemical NO donors and arginine rich diet, the results point toward an increase in all of the above factors, which result in the promotion and acceleration of the wound healing. Schaffer et al. J. Surg Res 63:237-240 (1996)

NO is also a known factor in promoting angiogenesis (development and rearrangement of new blood vessels within an injured tissue), increasing circulation to injured site, stimulating collagen synthesis in fibroblast, and mediating growth factor release. There are many situations a wound's healing response is delayed or inhibited in patients with systemic diseases. Systemic diseases include and are not limited to liver failure, renal impairment, diabetes, peripheral vascular disease, or in patients taking drugs like corticosteroids or immunosuppressive agents that inhibit healing, or prolonged process of healing in elderly. In all these cases, additional exogenous NO enhances the healing process. (Witte et al. Brit. J. Surg 89:1594-1601 (2002)

Keloid and hypertrophic scars are examples of scarring pathology that is characterized by excess collagen deposition during process of wound healing. The exact mechanism of this disorder is not well understood, but it is shown that NO expression and NO production are significantly reduced in fibroblasts derived from hypertrophic scars. By maintaining high levels of NO in these wounds, exogenous NO can offer a potential treatment.

There are many situations in which the healing response in a wound is delayed or inhibited in patients with systemic diseases. In all these cases, additional exogenous NO can potentially enhance or accelerate the wound healing process. One of these areas that NO can have vast therapeutic impact is patients with diabetes dealing with complicated non-healing wounds. As mentioned above, a systemic deficiency of endothelial-derived NO has been observed in diabetics, suggesting that NO plays a fundamental role in the pathogenesis of chronic, non-healing wounds. (Tas et al. Acta Vet. Brno 72:173-277 (2003)

In flap and micro-surgery reperfusion to ischemic tissue and organs is a critical criterion in survival of the tissue. Therefore administration of exogenous NO, due to its vasodilatory and angiogenesis effects, can potentially maintain the vascular tone and protect the skin flap.

Secondary infection in chronic and open wounds can seriously slow down or complicate the process of healing. NO antimicrobial has been well documented in literature. Nitric oxide has clearly shown bactericidal and/or bacteriostatic effects on at least two of the most common pathogens in chronic wounds, namely pseudomonas aeurginosa and staphylococcus aureus.

A number of molecules that produce NO under physiological conditions (NO donors) have been identified and evaluated both in vitro and in vivo. NO donor molecules exert biological effects mimicking those of NO and include S-nitrosothiols (Diodati et al, (1993) Thromb. Haem. 70:654-8; Leter et al, (1993) Circulation 88:2337-50; DeMeyer et al, (1995) J. Cardiovasc. Pharmacol. 26:272-9), organic nitrates (Ignarro et al, (1981) J. Pharmacol. Exp. Ther. 218:739-49), and complexes of NO with nucleophiles (Diodati et al, (1993) Thromb. Haem. 70:654-8; Diodati et al. (1993) J. Cardiovasc. Pharmacol. 22:287-92; Maragos et al, (1993) Cancer Res. 53:564-8).

Most of these have been low molecular weight molecules that are administered systemically and have short half-lives under physiologic conditions, thus exerting effects upon numerous tissue types with a brief period of activity. In addition, L-arginine is often thought as a NO donor, as L-arginine is a substrate for NO synthase, and thus administration of L-arginine increases endogenous NO production and elicits responses similar to those caused by NO donors in most cases (Cooke et al., (1992) J. Clin. Invest. 90:1168-72).

Disulfide, sulfide, sulfoxide and sulfone derivatives of dianhydrohexite mononitrate have been used or evaluated in various studies related to different pathological conditions mediated by defects in the NO pathway, such as cardiovascular disorders [WO00/20420 and WO2005/037842]. In spite of these studies, there has been no recognition that these compounds are capable of being effective as wounds healing.

### BRIEF DESCRIPTION OF THE INVENTION

The inventors have surprisingly found that compounds of formula (I), and specially 2-acetyl thioisosorbide-5-mononitrate, 2-[(R,S)-propylsulfinyl] isosorbide-5-mononitrate and 2-methylthioisosorbide 5-mononitrate, have potential therapeutic effect in wounds healing.

The new application of these compounds is based on the results obtained by *in* vivo experiments in animals subjected to removing of skin to cause a thickness wound, wherein upon administration of compounds of formula (I), wound contraction and wound re-epithelialization was observed. These experimental results have pointed out that the compounds of formula (I) allow an improved healing of wounds as well as a more reducing wound area when compared to other conventional medicaments, such as *Centella asiatica.*

Accordingly, the present invention relates to the use of a compound of formula (I) or a tautomer, a pharmaceutically acceptable salt, a prodrug or a solvate thereof: 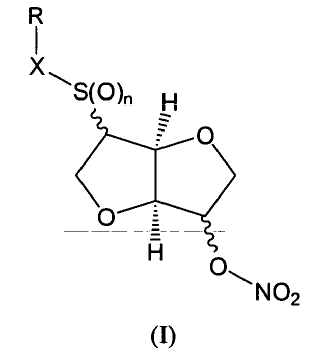 wherein:
n is an integer selected from 0, 1 and 2;
X is -S(O)ₘ-, -(C=O)- or a single bond, wherein m is an integer selected form 0, 1 and 2, with the proviso that when X is -(C=O)- then n is 0;
R is hydrogen or a residue R^{a}, wherein R^{a} is selected from the group consisting of:
   C₁₋₆ alkyl;
   C₂₋₆ alkenyl;
   C₃₋₈ cycloalkyl, wherein one CH₂ group is optionally replaced by O, S, NH or NCH₃;
   C₄₋₈ cycloalkenyl;
   phenyl;
   pyridyl;
   thiophenyl;
   nitrosyl;
   S-cysteinyl;
   S-glutathionyl; and 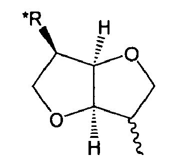 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen;
   and wherein R^{a} is optionally substituted by one to three groups independently selected from C₁₋₆ alkyl, C₂₋₆ alkenyl, C₃₋₈ cycloalkyl, C₄₋₈ cycloalkenyl, acetyloxy, hydroxyl, ONO₂ and halogen,
as active ingredient(s) in the manufacture of a pharmaceutical composition for wound healing.

In a particular embodiment, the wound is a foot ulcer, venous or pressure ulcer, post surgery hospital acquired infectious wounds, keloids, hypertrophic scarring, burns, sking flaps, non healing wounds in elderly, diabetic or immunocompromised patients. In a preferred embodiment, the wound is a physical injury to the body consisting of a laceration or breaking of the skin or mucous membrane.

In another aspect, the invention relates to a compound of formula (I) as defined above for its use in wound healing.

Finally, in another aspect, the present invention refers to a method for treating wounds comprising administering to a patient in need thereof a therapeutically effective amount of a compound of formula (I) as defined above or a tautomer, a pharmaceutically acceptable salt, a prodrug or a solvate thereof.

### DETAILED DESCRIPTION OF THE INVENTION

In the above definition of compounds of formula (I) used in the present invention, the following terms have the meaning indicated:
"C₁₋₆ alkyl" as used herein refers to a straight or branched hydrocarbon chain radical consisting of carbon and hydrogen atoms, containing no unsaturation, having one to six carbon atoms, and which is attached to the rest of the molecule by a single bond, e. g., methyl, ethyl, *n*-propyl, *i*-propyl, *n*-butyl, *t*-butyl, *n*-pentyl, etc.
"C₂₋₆ alkenyl" as used herein refers to a straight or branched chain alkenyl moiety consisting of carbon and hydrogen atoms, having one to six carbon atoms and at least one double bond of either E or Z stereochemistry where applicable, e.g., vinyl, allyl, 1- and 2-butenyl, and 2-methyl-2-propenyl.
"C₃₋₈ cycloalkyl" as used herein refers to an alicyclic group consisting of carbon and hydrogen atoms, having three to eight carbon atoms, e.g., cyclopropyl, cyclobutyl, cyclopentyl and cyclohexyl. Optionally, one CH₂ group of the cycloalkyl radical is replaced by O, S, NH o NCH₃, e.g., tetrahydropyrane, tetrahydrofurane, pyrrolidine, piperidine and tetrahydrothiophene.
"C₄₋₈ cycloalkenyl" as used herein refers to an alicyclic group consisting of carbon and hydrogen atoms, having four to eight carbon atoms, e.g., cyclopentenyl, cyclohexenyl, cycloheptenyl and cyclooctenyl.
"Halogen" as used herein refers to fluorine, chlorine, bromine or iodine, whereof bromine is preferred.

In a preferred embodiment, R represents hydrogen, C₁₋₆ alkyl, C₂₋₆ alkenyl, C₃₋₈ -cycloalkyl wherein one CH₂ group of the cycloalkyl radical is optionally replaced by O, S, NH o NCH3, C₄₋₈ cycloalkenyl, nitrosyl or the group: 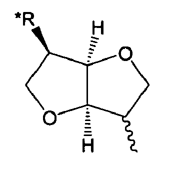 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen.

Even in a more preferred embodiment, R represents hydrogen, C₁₋₆ alkyl or the group: 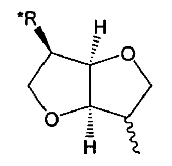 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen.

It is even more preferably that R represents C₁₋₆ alkyl.

It is further preferred that in formula (I) either one or both of m and n is 0.

It is especially preferred that in the compounds of formula (I), RXS(O)ₙ- and ONO₂ are trans to each other with respect to the ring plane. The compound of formula (I) also include (R) and (S) diastereoisomers according to the formula (Ia) and (Ib):

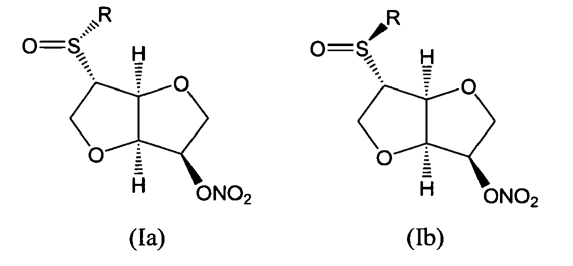

Especially preferred compounds of formula (I) are:

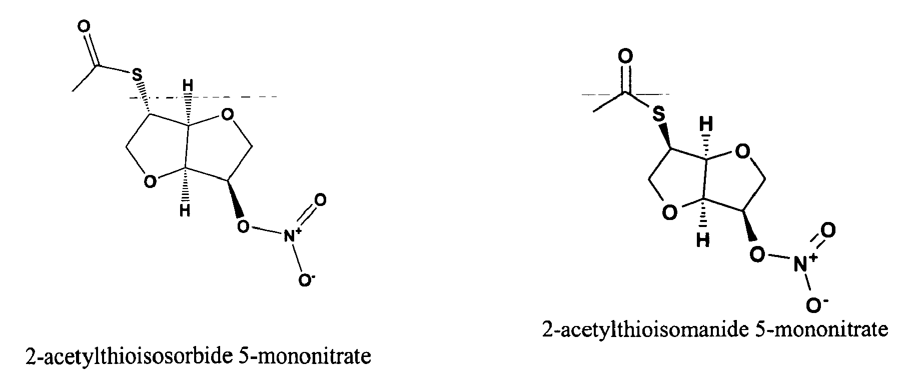

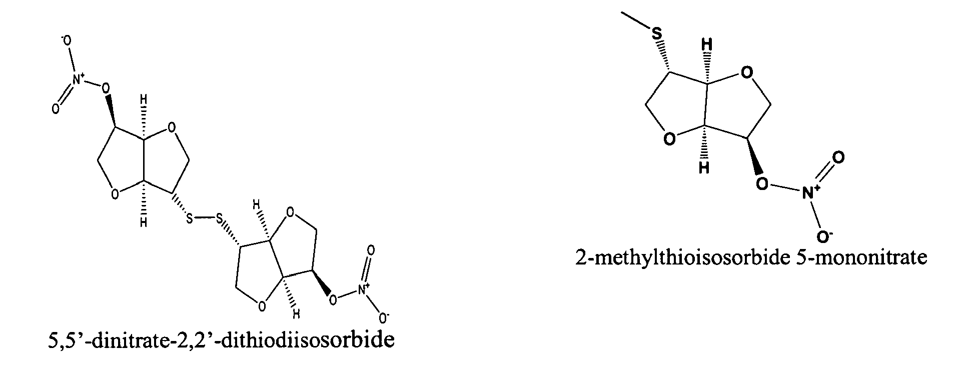

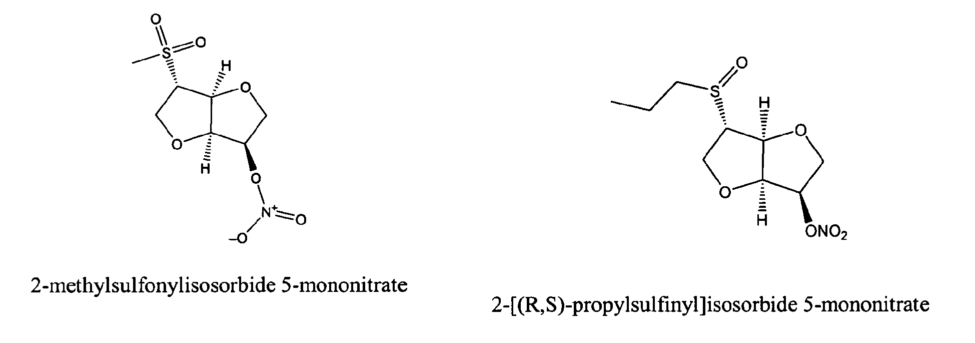

Further, it is especially preferred to use 2-acetyl thioisosorbide-5-mononitrate, 2-[(R,S)-propylsulfinyl] isosorbide-5-mononitrate and 2-methylthioisosorbide 5-mononitrate, even more preferably 2-acetyl thioisosorbide-5-mononitrate; a tautomer, a pharmaceutically acceptable salt, a prodrug or a solvate thereof as active ingredient for the manufacture of a pharmaceutical composition for the healing of wounds.

In a particular embodiment the wound is a physical injury to the body consisting of a laceration or breaking of the skin or mucous membrane.

Unless otherwise stated, the compounds used in the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The term "pharmaceutically acceptable salts, solvates, prodrugs" refers to any pharmaceutically acceptable salt, ester, solvate, or any other compound which, upon administration to the recipient is capable of providing (directly or indirectly) a compound as described herein.

For instance, pharmaceutically acceptable salts of compounds used in the invention are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

Particularly favoured derivatives or prodrugs are those that increase the bioavailability of the compounds of this invention when such compounds are administered to patient. (e.g., by enhancing delivery of the parent compound to a biological compartment relative to the parent species)

The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Text book of Drug design and Discovery" Taylor & Francis (April 2002).

The compounds used in the present invention may be in crystalline form either as free compounds or as solvates (e.g. hydrates).

The compounds of formula (I) or their salts or solvates used in the invention are preferably in pharmaceutically acceptable or substantially pure form. By pharmaceutically acceptable form is meant, "inter alia", having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

The compounds used in the present invention represented by the formula (I) can include enantiomers, depending on the presence of chiral centers, and/or depending on the presence of multiple bonds (for example Z, E). The pure isomers, enantiomers or diastereoisomers and their mixtures are within the scope of the present invention.

The compounds of formula (I) used in the invention can be obtained by available synthetic procedures. Some examples of these procedures are described in WO2005/037842 and references therein. The content of these documents is incorporated herein by reference in its entirety.

In a particular embodiment of the present invention, the compounds of formula (I) used in wound healings, are formulated in a suitable pharmaceutical composition, in a therapeutically effective quantity, together with one or more pharmaceutically acceptable carriers, adjuvants or excipients.

The pharmaceutical composition may be administered in the form of different topical preparations, e. g. solutions, suspensions, creams, ointments or pastes, gels, transdermic preparations such as patches or plasters. The pharmaceutical composition may also be prepared for vaginal or for rectal administration, e.g. rectal gel or suppository.

Generally an effective administered amount of a compound used in the invention will depend on the relative efficacy of the compound chosen, the severity of the disorder being treated and the weight of the patient. However, active compounds will typically be administered once or more times a day for example 1, 2, 3 or 4 times daily.

The compounds used in the present invention may also be administered with other drugs to provide a combination therapy. The other drugs may form part of the same composition, or be provided as a separate composition for administration at the same time or at different time. An example of a drug which can be used in combination with the compounds of formula (I) is Centella asiatica.

In a particular embodiment the invention refers to the use of a compound of formula (I) or a tautomer, a pharmaceutically acceptable salt, a prodrug or a solvate thereof, for the elaboration of a pharmaceutical composition to heal wounds.

### EXAMPLES

### Example 1.

### MATERIALS AND METHODS

### Animals

Male Wistar rats provided by Janvier, Espana weighing 150 - 200 g were housed in our animal facilities in groups of 3 animals, being each group in a conventional animal facility where the environmental temperature and relative humidity are monitored and controlled. The lighting schedule in the animal facility is 12 hours light and 12 hours dark. The rats were used after an acclimatization period of five days. After a week quarantine period animals were dosed.

### Preparation of hydrogels

Symperonic F-127 gels containing compounds of formula (I) were prepared by the "cold" method. Briefly, 1.63 g of Symperonic F-127 was added to 2.2 ml of cold (5°C) stirred water. The solutions were allowed to attain dissolution equilibrium for a period of 8 h. The appropriate amount of compound of formula (I) was added to the hydrogel solution for homogenization. Control hydrogel was prepared by the same procedure without addition of the active compound. The hydrogels were used immediately after preparation. Application of cold liquid F-127 solutions on the skin leads to rapid gelation.

### Treatments

Rats were anesthetized with sodium pentobarbital (40 mg/kg, i.p.) before treatments application to keep the animal calm to allow absorption of the products.

The dorsal surface was shaved and a full thickness wound (2 x 2 cm) was made on the back of each rat by removing the skin (epidermis and dermis) and exposing paniculus carnosus.

Groups of 5 rats for treatment were used, and 0.5 g of hydrogel was topically applied in a wound (2 x 2 cm) on the back of each rat.

Treatments were applied from the first day until fourth day after wounding. After application of hydrogel, (with or without active compound), wounds were covered by parafilm and gauze, that were fixed with adhesive tape. From the fifth day post-wounding, lesions were left uncovered in all groups. The animals were housed in individual cages with free access to food and water.

To evaluate wound contraction and wound re-epithelialization, lesion tracings were performed on a transparent sheet in the day of lesion and 1, 3, 5, 7 days after wounding.

The area of the wounds (cm²) and % re-ephitelización were estimated by the difference between the total lesion area and the wound area still uncovered.

In order to evaluate possible systemic circulatory effects during the topical application of products containing hiydrogel the bloodless determination of the mean arterial pressure (mm Hg) and heart rate (beats/min) was measured by means of sphygmomanometric sleeve (LE 5002, Letica, Panlab, SL) placed on the tail of the conscious rats following 30 min of treatment.
The following compounds were assayed:
Compound 1: 2-acetylthioisosorbide 5-mononitrate
Compound 2: 2-acetylthioisomanide 5-mononitrate
Compound 3: 5'5'-dinitrato-2,2'ditioisosorbide
Compound 4: 2-Methylthioisosorbide 5-mononitrate
Compound 5: 2-Methylsulfonylisosorbide 5-mononitrate
Compound 6: 2-[(R,S)-propylsulfinyl]isosorbide 5-mononitrate
Compound 7 (comparative example): Isosorbide 5-mononitrate (IS-5MN)
Compound 8 (comparative example): Centella asiática

### Results

The efficacy of treatments was evaluated by measuring wound area and percentage of re-epithelialization vs total wound area. Results are given in Table I.

**Table I**

| ***Treatment*** | **% *Re-epithelialization*** ***(day 5)*** | ***Wound area (cm²)*** ***(day 8)*** | ***% Wound reduction vs control*** |
|---|---|---|---|
| Control | 60 | 3.5 | - |
| Compound 1 | 100 | 1.2 | 66 |
| Compound 2 | 100 | 1.4 | 60 |
| Compound 3 | 100 | 1.7 | 51 |
| Compound 4 | 100 | 1.1 | 69 |
| Compound 5 | 90 | 1.7 | 51 |
| Compound 6 | 90 | 1.1 | 69 |
| Compound 7 (IS-5MN) | 60 | 1.4 | 60 |
| Comparative example Compound 8 *(Centella asiatica)* Comparative example | 60 | 2.7 | 22 |

These experimental results have pointed out that the compounds of formula (I) allow an improved and hastened healing of wounds as well as a more reducing wound area when compared to other conventional medicaments, such as *Centella asiatica* or isosorbide 5-mononitrate.

No systemic effect of any compound was observed after measuring arterial blood pressure (mm Hg) and heart rate (beats/mean) before and during 30 minutes after application.

## Claims

1. Use of a compound of formula (I) or a tautomer, a pharmaceutically acceptable salt, a prodrug or a solvate thereof: 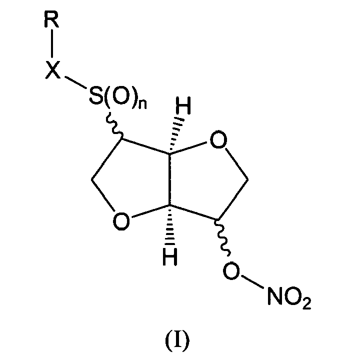 wherein:
n is an integer selected from 0, 1 and 2;
X is -S(O)ₘ-, -(C=O)- or a single bond, wherein m is an integer selected form 0, 1 and 2, with the proviso that when X is -(C=O)- then n is 0;
R is hydrogen or a residue R^{a}, wherein R^{a} is selected from the group consisting of:
C₁₋₆ alkyl;
C₂₋₆ alkenyl;
C₃₋₈ cycloalkyl;
C₃₋₈ cycloalkyl, wherein one CH₂ group is replaced by O, S, NH or NCH₃;
C₄₋₈ cycloalkenyl;
phenyl;
pyridyl;
thiophenyl;
nitrosyl;
S-cysteinyl;
S-glutathionyl; and 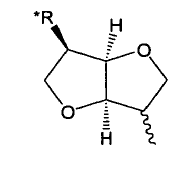 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen;
and wherein R^{a} is optionally substituted by one to three groups independently selected from C₁₋₆ alkyl, C₂₋₆ alkenyl, C₃₋₈ cycloalkyl, C₄₋₈ cycloalkenyl, acetyloxy, hydroxyl, ONO₂ and halogen,
as active ingredient(s) in the manufacture of a pharmaceutical composition for wound healing.

2. The use according to claim 1, wherein the wound is a foot ulcer, venous or pressure ulcer, post surgery hospital acquired infectious wounds, keloids, hypertrophic scarring, bums, sking flaps, non healing wounds in elderly, diabetic, or immunocompromised patients.

3. The use according to claim 1, wherein the wound is a physical injury to the body consisting of a laceration or breaking of the skin or mucous membrane.

4. The use according to any one of claims 1 to 3, wherein at least one of m and n is 0.

5. The use according to any one of claims 1 to 3, wherein X represents a single bond or -S-.

6. The use according to any one of claims 1 to 3, wherein X represents a C=O group.

7. The use according to any one of claims 1 to 6, wherein R is hydrogen, C₁₋₆ alkyl, C₂₋₆ alkenyl, C₃₋₈ cycloalkyl wherein one CH₂ group of the cycloalkyl radical is optionally replaced by O, S, NH o NCH₃, C₄₋₈ cycloalkenyl, nitrosyl or the group: 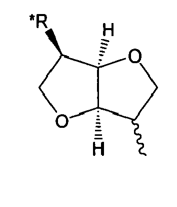 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen.

8. The use according to claim 7, wherein R is hydrogen, C₁₋₆ alkyl or the group: 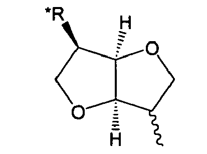 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen.

9. The use according to any one of claims 7 or 8, wherein R is C₁₋₆ alkyl.

10. The use according to any one of claims 1 to 9, wherein the compound of formula (I) is a compound of formula (Ia) or (Ib):

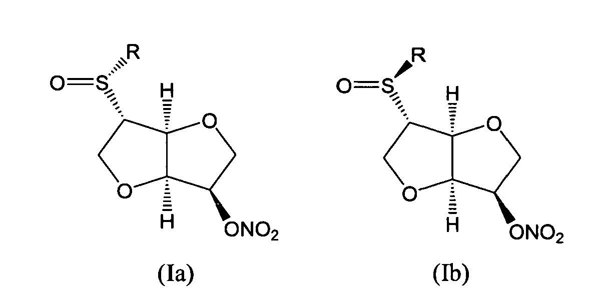

11. The use according to claim 1 or 10, wherein the compound of formula (I) is selected from:
2-acetylthioisosorbide 5-mononitrate
2-acetylthioisomanide 5-mononitrate
5'5'-dinitrato-2,2'ditioisosorbide
2-Methylthioisosorbide 5-mononitrate
2-Methylsulfonylisosorbide 5-mononitrate
2-[(R,S)-propylsulfinyl]isosorbide 5-mononitrate

12. The use according to claim 11, wherein the compound of formula (I) is selected from 2-acetyl thioisosorbide-5-mononitrate, 2-[(*R*,*S*)-propylsulfinyl] isosorbide-5-mononitrate and 2-methylthioisosorbide 5-mononitrate.

13. A compound of formula (I) as defined in any of claims 1 or 4 to 12, for its use in wound healing.

14. The compound according to claim 13, wherein the wound is a foot ulcer, venous o pressure ulcer, post surgery hospital acquired infectious wounds, keloids, hypertrophic scarring, bums, sking flaps, non-healing wounds in elderly, diabetic or immunocompromised patients.

15. The compound according to claim 13, wherein the wound is a physical injury to the body consisting of a laceration or breaking of the skin or mucous membrane.
